# EUROPEAN PATENT APPLICATION

(11) **EP 4 344 700 A1**
(43) Date of publication of application: **03.04.2024**
(21) Application number: 22215747.1
(22) Date of filing: 22.12.2022
(51) Int. Cl.: A61K 31/404, A61K 45/00, A61N 5/06, A61P 17/06, C12N 15/00, C07K 14/47, C07K 14/475, C07K 14/49, C07K 14/54

(54) **VEGFR GENE EXPRESSION INHIBITOR FOR USE IN THE TREATMENT OF PSORIASIS**

(30) Priority: 28.09.2022 CN 202211189318
(71) Applicant: Sun, Liangdan, Hefei City, Anhui 230022 (CN)
(72) Inventor: SUN, Liangdan, Hefei City, Anhui (CN); LI, Zhuo, Hefei City (CN); ZHEN, Qi, Hefei City (CN); CHEN, Weiwei, Hefei City (CN); WANG, Yirui, Hefei City (CN)
(74) Representative: Herrero & Asociados, S.L.

(57) **Abstract**

An inhibitor of vascular endothelial growth factor receptor (VEGFR) gene expression for use in treating psoriasis is provided. Also provided is a narrow-band ultraviolet B (NBUVB) radiation that effectively inhibit an abnormal expression of a renin-angiotensin system guanyl releasing protein 1 (RASGRPI) gene in skin T-lymphocytes, thereby inhibiting the T-lymphocytes generating interleukin-17 (IL-17), and thus effectively alleviating IL-17-mediated immune response of the psoriasis in skin sites to achieve an effect of treating the psoriasis.

## Description

### TECHNICAL FIELD

The invention relates to a technical field of medicines for skin diseases, in particular to an application of vascular endothelial growth factor receptor (VEGFR) gene expression inhibitor in preparing a preparation or as a preparation for treating psoriasis.

### BACKGROUND

Psoriasis is a chronic immune inflammatory skin disease characterized by excessive proliferation and abnormal differentiation of epidermal keratinocytes, infiltration of lymphocytes (mostly T-lymphocytes, also referred to as T cells), and changes of dermal blood vessels, such as angiogenesis, vascular dilatation, vascular tortuosity, and formation of high endothelial vessels. Psoriasis vulgaris is the most common clinical manifestation of psoriasis. Psoriasis has a wide range of etiologies and complex pathogenesis, and immune pathway plays an important role in the pathogenesis of psoriasis. The pathogenesis of psoriasis is closely related to chronic inflammation mediated by abnormal activation of the T cells. Studies have shown that the psoriasis is caused by an activation of the T cells secreted with pro-inflammatory cytokines, including tumor necrosis factor-alpha (TNF-α), interleukin-17A (IL-17A) and interferon-gamma (IFN-γ). The existence and accumulation of the T cells is an initial phenomenon in an evolution of psoriatic plaques. The abnormal activation of the T cells can affect skin immunity and systemic cytokine production, thereby stimulating a recruitment of more inflammatory cells to induce the characteristic epidermal changes of the psoriasis.

At present, the clinical use of biological agents targeting IL17A/F has a good therapeutic effect on psoriasis, but the related biological agents are expensive and require to be continuously used at a later stage, which makes treatment cost is high and the psoriasis cannot be eradicated.

Therefore, a safe and effective preparation for treating psoriasis is necessary.

### SUMMARY

In response to the above problems, in a first aspect, the invention aims to provide an application of a vascular endothelial growth factor receptor (VEGFR) gene expression inhibitor in preparing a preparation or as a preparation for treating psoriasis. Specifically, the invention effectively inhibits interleukin-17 (IL-17) generated by T cells through inhibiting VEGFR gene expression, thereby effectively treating the psoriasis. It needs to be noted that the invention is based on a mechanism of inhibiting the VEGFR gene expression and thereby reducing or eliminating the psoriasis. The VEGFR gene expression inhibitors used in the invention are all the reagents known in the related art that can inhibit VEGFR gene expression.

In a second aspect, the invention aims to provide an application of a rat sarcoma guanyl releasing protein 1 (RASGRP1) gene expression inhibitor in preparing a preparation or as a preparation for treating the psoriasis. Specifically, the invention effectively inhibits interleukin-17 (IL-17) generated by the T cells by inhibiting RASGRP1 gene expression, thereby effectively treating the psoriasis. Similarly, as described above, the invention is based on a mechanism of inhibiting the RASGRP1 gene expression and thereby reducing or eliminating the psoriasis.

In an illustrated embodiment of the invention, the preparation of the application described above includes a chemical reagent or a biological reagent. For example, the VEGFR gene expression inhibitors can be chemical reagents such as small molecule VEGFR gene inhibitors, or reagents for genome editing that knock down or knock out the VEGFR gene, such as CRISPR-Cas reagents (referred to as reagents used in clustered regularly interspaced palindromic repeats (CRISPR) associate system), transcription activator-like effector nuclease (TALEN) reagents, or zinc finger nucleases (ZFN) reagents. Similarly, the RASGRP1 gene expression inhibitors can be small molecule chemical reagents that can inhibit RASGRP1 gene expression, or reagents that can be used to knock out or knock down the RASGRP1 gene, such as CRISPR-Cas reagents, TALEN reagents, or ZFN reagents.

In an illustrated embodiment of the invention, the preparation of the application described above is in a form of an ointment, powder, or an aqueous solution. It should be noted that the VEGFR gene expression inhibitor or the RASGRP1 gene expression inhibitor can be added with excipients to make various formulations suitable for the clinical use. Furthermore, the VEGFR gene expression inhibitor or the RASGRP1 gene expression inhibitor can be used in combination with other reagents for treating the psoriasis.

In a third aspect, the invention aims to provide an application of narrow-band ultraviolet B (NBUVB) radiation to inhibit an expression of a VEGF gene.

In a fourth aspect, the invention aims to provide an application of NBUVB radiation to inhibit an expression of a RASGRP1 gene.

In a fifth aspect, the invention aims to provide an application of NBUVB radiation to inhibit IL-17 generated by T cells.

It should be noted that the NBUVB radiation can inhibit the expression of the VEGF gene in human body to thereby inhibit the expression of the RASGRP1 gene, and the inhibition of the RASGRP1 gene expression can effectively inhibit IL-17 secreted by downstream T cells, thus effectively treating the psoriasis and achieving a precise treatment of the psoriasis. In addition, the invention constructs a mouse model with successful psoriasis treatment by the NBUVB radiation and performs ribonucleic-acid sequencing (RNA-Seq) pathway enrichment analysis to find that the NBUVB radiation can significantly inhibit rat sarcoma (RAS) pathway, which enriches the pathogenesis of the NBUVB radiation for treating psoriasis and provides a theoretical basis for gene diagnosis, targeted therapy, and new drug research and development of the psoriasis.

In a sixth aspect, the invention aims to provide a method for inhibiting T cells generating IL-17, which includes NBUVB radiation, or knocking out or knocking down a VEGF gene, or knocking out or knocking down a RASGRP1 gene. Specifically, as described above, the invention effectively inhibits the T cells generating the IL-17 by the NBUVB radiation; the invention inhibits the T cells generating the IL-17 by inhibiting the VEGF gene expression; or the invention inhibits the T cells generating the IL-17 by inhibiting the RASGRP1 gene expression.

It should also be noted that the psoriasis, as a complex disease, is not influenced by a single factor. Therefore, the search for the intrinsic mechanisms of disease onset and regulation and weight of various influencing factors in the disease is the basis and prerequisite for efficient selection of disease targets in the future. Vascular endothelial growth factor is an important angiogenesis-promoting factor with an ability to promote increased vascular permeability and induce angiogenesis. In the invention, the NBUVB radiation can significantly inhibit the VEGF gene expression in the skin tissue of the mouse with the psoriasis.

It should also be noted that the RASGRP1 is a rat sarcoma guanine nucleotide exchange factor, which is an important regulatory factor of lymphocyte receptor signaling. In addition, the RASGRP1 gene is one of the downstream target genes of the VEGF gene and plays an important role in growth and development of the T cells. The RASGRP1 gene regulates T cells/B-cells development, homeostasis, and differentiation by coupling T-lymphocyte/B-lymphocyte antigen receptors to the RAS. A necessary signal for the abnormal activation of the T cells is a recognition signal of T-cell receptor (TCR). When the T cells are stimulated to activate the TCR, the expression of the RASGRP1 gene increases, namely that the RASGRP1 gene is with high expression in the T cells. The high expression of the RASGRP1 gene plays an important role in activating and transmitting the TCR signal, which is acting as a bridge to transmit the signal to the downstream to promote a proliferation and activation of the T cells, and the activated T cells secrete various cytokines and inflammatory mediators to mediate the occurrence and development of the psoriasis.

The beneficial effects of the invention include that the invention can effectively inhibit the abnormal expression of the RASGRP1 gene in the skin T cells by the NBUVB radiation, thereby inhibiting the T cells secreting the IL-17, thus effectively alleviating IL-17-mediated immune response in psoriatic lesions to achieve an effect of treating psoriasis.

### BRIEF DESCRIPTION OF DRAWINGS

FIG. 1 shows skin pathological phenotypes of psoriatic mice with narrow-band ultraviolet B (NBUVB) radiation.
FIGS. 2A-2D show psoriasis area and severity index (PASI) scores of the psoriatic mice with NBUVB radiation.
FIG. 3 shows pathways enrichment by a transcriptome sequencing (also referred to as RNA sequencing), revealing that the NBUVB radiation inhibits a rat sarcoma (RAS) pathway.
FIGS. 4A-4H show detection results of messenger ribonucleic-acid (mRNA) in the RAS pathway in skin tissues of the psoriatic mice with NBUVB radiation by real-time fluorescence quantitative polymerase chain reaction (PCR).
FIG. 5 shows protein expressions in the RAS pathway in the skin tissues of the psoriatic mice with NBUVB radiation.
FIG. 6 shows results of renin-angiotensin system guanyl releasing protein 1 (RASGRP1) +T cells and interleukin-17A (IL-17A) +T cells of the psoriatic mice with NBUVB radiation detected by flow cytometry.
FIGS. 7A-7F show statistics results, represented by a form of histogram, of the RASGRP1+T cells and the IL-17A+T cells of the psoriatic mice with NBUVB radiation detected by the flow cytometry.
FIG. 8 shows skin pathological phenotypes of an imiquimod-induced psoriatic mice model.
FIG. 9 shows an expression and a location of RASGRP1 in skin tissues of the imiquimod-induced psoriatic mice model.
FIGS. 10A-10H show detection results of mRNA in RAS pathway in the skin tissues of the imiquimod-induced psoriatic mice model by real-time fluorescence quantitative PCR.
FIG. 11 shows protein expressions in the RAS pathway in the skin tissues of the imiquimod-induced psoriatic mice model.
FIG. 12 shows results of RASGRP1+T cells, RASGRP1+Monocytes and RASGRP1+Neutrophils in the skin tissues of the imiquimod-induced psoriatic mice model detected by flow cytometry.
FIGS. 13A-13C show statistics results, represented by a form of histogram, of the RASGRP1+T cells, the RASGRP1+Monocytes and the RASGRP1+Neutrophils in the skin tissues of the imiquimod-induced psoriatic mice model detected by the flow cytometry.
FIG. 14 shows T cells and RASGRP1 in the skin tissues of the imiquimod-induced psoriatic mice model detected by double-labeling immunohistochemistry.
FIG. 15 shows detection results of mRNA of VEGF in the skin tissues of the psoriatic mice with NBUVB radiation by the real-time fluorescence quantitative PCR.
FIG. 16 shows an expression and a location of VEGF in the skin tissues of the psoriatic mice with NBUVB radiation.
FIG. 17 shows detection results of mRNA of VEGF in epidermal tissues of the psoriatic mice with NBUVB radiation by the real-time fluorescence quantitative PCR.
FIG. 18 shows a protein expression of VEGF in the epidermal tissues of the psoriatic mice with NBUVB radiation.
FIG. 19 shows detection results of mRNA of VEGF in dermal tissues of the psoriatic mice with NBUVB radiation by the real-time fluorescence quantitative PCR.
FIG. 20 shows a protein expression of VEGF in the dermal tissues of the psoriatic mice with NBUVB radiation.
FIGS. 21A-21B show results indicating main cell populations expressed with vascular endothelial growth factor A (VEGFA) in human psoriatic lesions by single-cell sequencing.
FIGS. 22A-22C show changes of VEGF concentration in cell supernatants extracted from immortalized nontumorigenic human epidermal (HaCaT) cells after treated with NBUVB radiation in different radiation dose gradients and different radiation time gradients.
FIG. 23 shows VEGF concentrations, detected by enzyme-linked immunosorbent assay (ELISA), in cell supernatants extracted from HaCaT cells treated with NBUVB radiation, HaCaT cells treated without NBUVB radiation, and HaCaT cells stimulated by IL-17A and tumor necrosis factor alpha (TNF-α) and treated with NBUVB radiation, and HaCaT cells stimulated by IL-17A and TNF-α cytokines and treated with NBUVB radiation respectively.
FIG. 24 shows skin pathological phenotypes of psoriatic mice subcutaneously injected with VEGF.
FIGS. 25A-25D show PASI scores of the psoriatic mice subcutaneously injected with VEGF.
FIG. 26 shows protein expressions in RAS pathway in skin tissues of the psoriatic mice subcutaneously injected with VEGF.
FIG. 27 shows results of RASGRP1+T cells and IL-17A+T cells in the skin tissues of the psoriatic mice subcutaneously injected with VEGF detected by flow cytometry.
FIGS. 28A-28F show statistics results, represented by a form of histogram, of the RASGRP1+T cells and the IL-17A+T cells in the skin tissues of the psoriatic mice subcutaneously injected with VEGF detected by the flow cytometry.
FIG. 29 shows skin pathological phenotypes of psoriatic mice with skin smeared with VEGFR small molecular inhibitor SU5205.
FIGS. 30A-30D show PASI scores of the psoriatic mice with skin smeared with VEGFR small molecular inhibitor SU5205.
FIG. 31 shows protein expressions in RAS pathway in skin tissues of the psoriatic mice with skin smeared with VEGFR small molecular inhibitor SU5205.
FIG. 32 shows results of RASGRP1+T cells and IL-17A+T cells in the skin tissues of the psoriatic mice with skin smeared with VEGFR small molecular inhibitor SU5205 detected by flow cytometry.
FIGS. 33A-33F show statistics results, represented by a form of histogram, of the RASGRP1+T cells and the IL-17A+T cells in the skin tissues of the psoriatic mice with skin smeared with VEGFR small molecular inhibitor SU5205 detected by the flow cytometry.
FIGS. 34A-34C show detection results of mRNA of IL1b (also referred to as IL-1β) and IL17 in Jurkat cells with RASGRP1 overexpression stimulated by VEGF by real-time fluorescence quantitative PCR.
FIGS. 35A-35B show concentrations of the IL1b and the IL17 in cell supernatants extracted from the Jurkat cells with RASGRP1 overexpression stimulated by VEGF.
FIG. 36 shows protein expressions in RAS pathway of the Jurkat cells with RASGRP1 overexpression stimulated by VEGF.
FIGS. 37A-37C show detection results of mRNA of IL1b and IL17 in Jurkat cells with RASGRP1 knockdown stimulated by VEGF by real-time fluorescence quantitative PCR.
FIGS. 38A-38B show concentrations of the IL1b and the IL17 in cell supernatants extracted from the Jurkat cells with RASGRP1 knockdown stimulated by VEGF.
FIG. 39 shows protein expressions in RAS pathway of the Jurkat cells with RASGRP1 knockdown stimulated by VEGF.
FIG. 40 shows skin pathological phenotypes of psoriatic mice subcutaneously injected with RASGRP1 overexpression adeno-associated virus.
FIGS. 41A-41D show PASI scores of the psoriatic mice subcutaneously injected with RASGRP1 overexpression adeno-associated virus.
FIG. 42 show concentrations of IL-1β in skin tissues of the psoriatic mice subcutaneously injected with RASGRP1 overexpression adeno-associated virus.
FIGS. 43A-43H show detection results of mRNA in RAS pathway in the skin tissues of the psoriatic mice subcutaneously injected with RASGRP1 overexpression adeno-associated virus by real-time fluorescence quantitative PCR.
FIG. 44 shows protein expressions in the RAS pathway in the skin tissues of the psoriatic mice subcutaneously injected with RASGRP1 overexpression adeno-associated virus.
FIG. 45 shows results of RASGRP1+T cells and IL-17A+T cells in the skin tissues of the psoriatic mice subcutaneously injected with RASGRP1 overexpression adeno-associated virus detected by flow cytometry.
FIGS. 46A-46F show statistics results, represented by a form of histogram, of the RASGRP1+T cells and the IL-17A+T cells in the skin tissues of the psoriatic mice subcutaneously injected with RASGRP1 overexpression adeno-associated virus detected by the flow cytometry.
FIG. 47 shows skin pathological phenotypes of psoriatic mice with RASGRP1 overexpression treated with NBUVB radiation.
FIGS. 48A-48D show PASI scores of the psoriatic mice with RASGRP1 overexpression treated with NBUVB radiation.
FIG. 49 shows concentrations of IL-1β in skin tissues of the psoriatic mice with RASGRP1 overexpression treated with NBUVB radiation.
FIGS. 50A-50H show detection results of mRNA in RAS pathway in the skin tissues of the psoriatic mice with RASGRP1 overexpression treated with NBUVB radiation by real-time fluorescence quantitative PCR.
FIG. 51 shows protein expressions in the RAS pathway in the skin tissues of the psoriatic mice with RASGRP 1 overexpression treated with NBUVB radiation.
FIG. 52 shows results of RASGRP1+T cells and IL-17A+T cells in the skin tissues of the psoriatic mice with RASGRP1 overexpression treated with NBUVB radiation detected by flow cytometry.
FIGS. 53A-53F show statistics results, represented by a form of histogram, of the RASGRP1+T cells and the IL-17A+T cells in the skin tissues of the psoriatic mice with RASGRP1 overexpression treated with NBUVB radiation detected by the flow cytometry.
FIG. 54 shows skin pathological phenotypes of RASGRP1-/- psoriatic mice.
FIGS. 55A-55D show PASI scores of skin tissues of the RASGRP1-/- psoriatic mice.
FIG. 56 shows concentrations of IL-1β in the skin tissues of the RASGRP1-/- psoriatic mice.
FIGS. 57A-57G show detection results of mRNA in RAS pathway in the skin tissues of the RASGRP1-/- psoriatic mice by real-time fluorescence quantitative PCR.
FIG. 58 shows protein expressions in the RAS pathway in the skin tissues of the RASGRP1-/- psoriatic mice.
FIG. 59 shows results of IL-17A+T cells in the skin tissues of the RASGRP1-/- psoriatic mice detected by flow cytometry.
FIGS. 60A-60C show statistics results, represented by a form of histogram, of the IL-17A+T cells in the skin tissues of the RASGRP1-/- psoriatic mice detected by the flow cytometry.

### DETAILED DESCRIPTION OF THE EMBODIMENTS

Embodiments are given to better illustrate the invention, but the invention is not limited by the embodiments. Therefore, non-essential improvements and adjustments to the embodiments made by those skilled in the related art according to the above descriptions of the invention still fall within the scope of the protection of the invention.

The terms used herein are only intended to describe the illustrated embodiments and are not intended to limit the invention. Unless the context has specific explanation, expressions in a singular form include expressions in a plural form. As used herein, the terms such as "includes", "has", "contains", and the like are intended to indicate features, numbers, operations, components, parts, elements, materials, or combination thereof. The terms of the invention are disclosed in the specification and are not intended to exclude the possibility that one or more other features, numerals, operations, components, parts, elements, materials, or combinations thereof may be present or may be added. As used herein, "/" may be interpreted as "and" or "or", as the case may be.

In order to better illustrate the invention, the invention is further described in combination with specific embodiments, but the invention is not only limited to the following embodiments.

In the following embodiments, the experimental operations in the invention without specific description are conventional experimental operation methods in the related field.

### Embodiment 1 alleviation of phenotype of psoriatic mice achieved by NBUVB radiation to inhibit activation of RAS downstream pathways in skin tissues

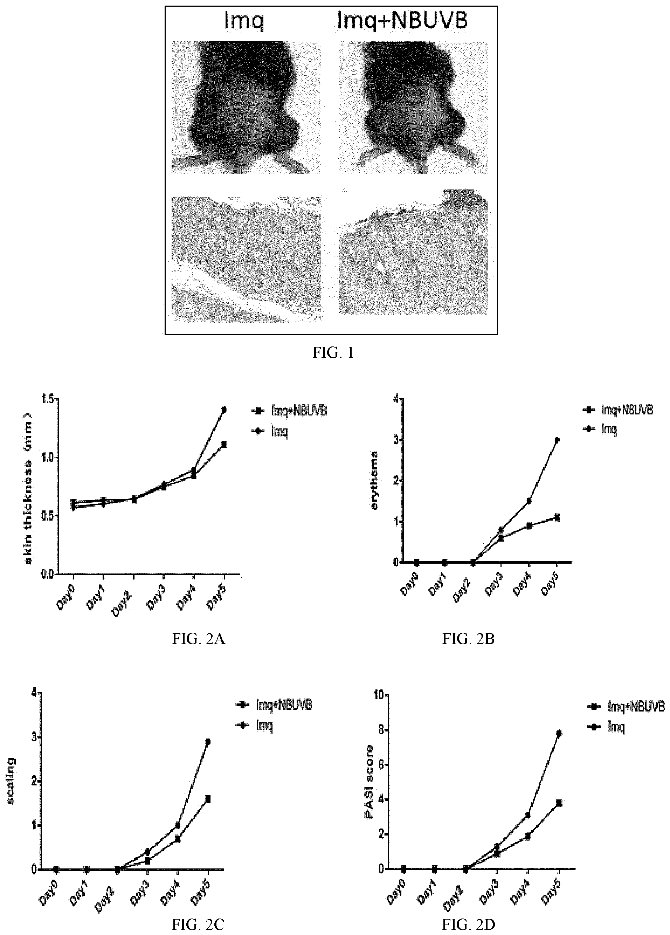

In the embodiment of the invention, the psoriatic mice are treated with NBUVB radiation, and the NBUVB radiation can reduce skin inflammatory of the psoriatic mice. Hematoxylin-eosin (H&E) staining shows a significant thinning of skin epidermal tissues and a reduction of inflammatory cell infiltration, which indicates an alleviation of inflammation (as shown in FIG. 1).

In addition, PASI scores are also performed on skin tissues of the psoriatic mice, which reflect reductions of dorsal skin thickness, erythema, and scaling (as shown in FIGS. 2A-2D).

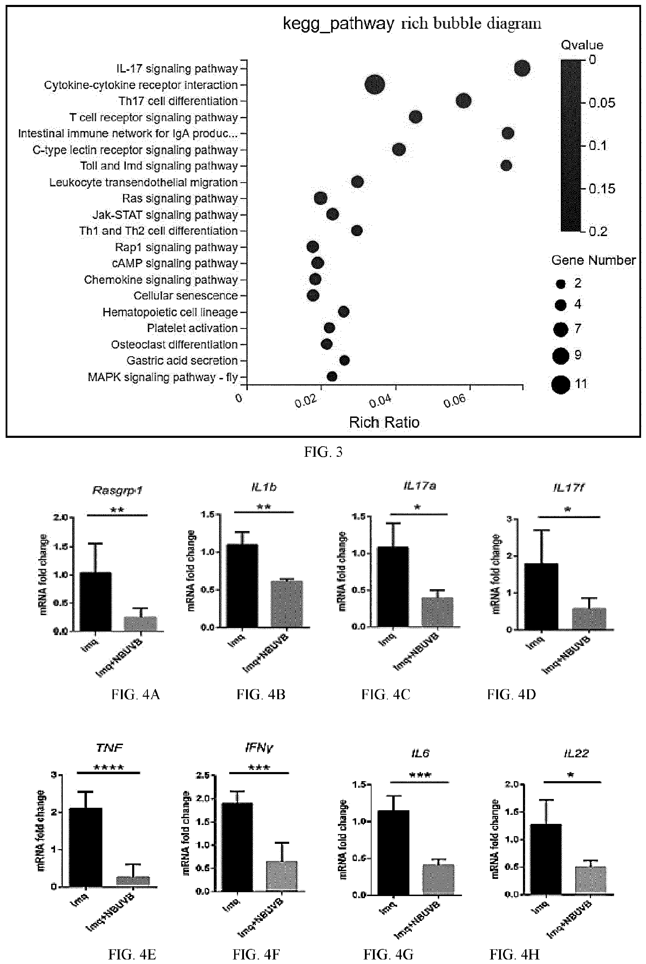

In addition, enrichment analysis of pathways is performed by RNA sequencing, which shows that the RAS pathway is significantly inhibited after the NBUVB radiation (as shown in FIG. 3).

In addition, real time quantitative-Polymerase Chain Reaction (RT-qPCR) is used to detect RASGRP1 gene, and relative downstream cytokines IL1b, IL17a, etc. The results show that expression levels of the inflammatory genes such as the RASGRP1 gene, and the relative downstream cytokines IL1b, IL17a in the skin tissues of the psoriatic mice treated with the NBUVB radiation are significantly decreased (as shown in FIGS. 4A-4H).

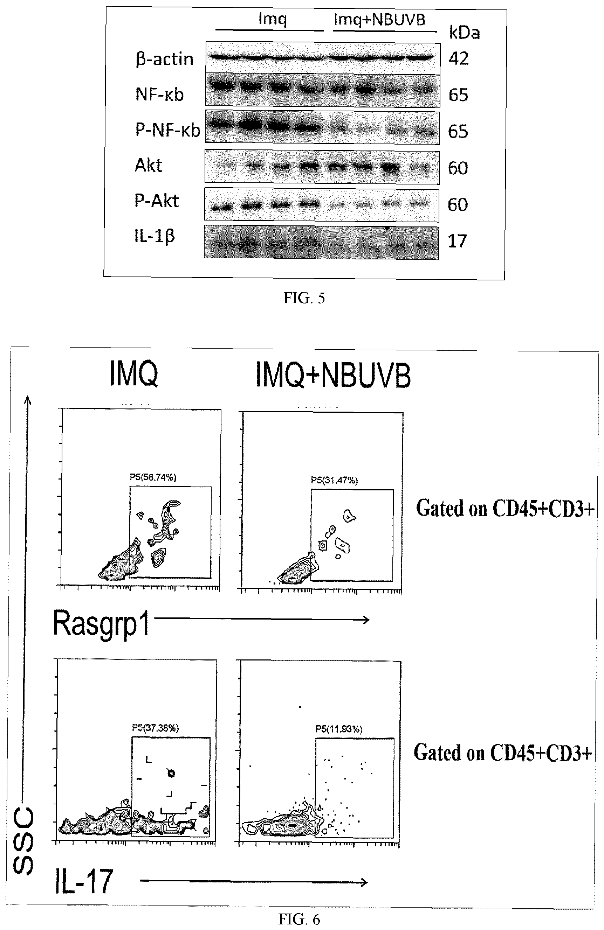

In addition, Western blot detection is used to detect protein expressions of RASGRP1 downstream pathways. The results show that active protein levels of nuclear factor kappa-B (NF-κB) and phosphorylated AKT (also referred to as Protein kinase B) in the skin tissues of the psoriatic mice treated with the NBUVB radiation are lower than those of control mice, among which the active protein level of IL-1β is also lower than that of the control mice (as shown in FIG. 5).

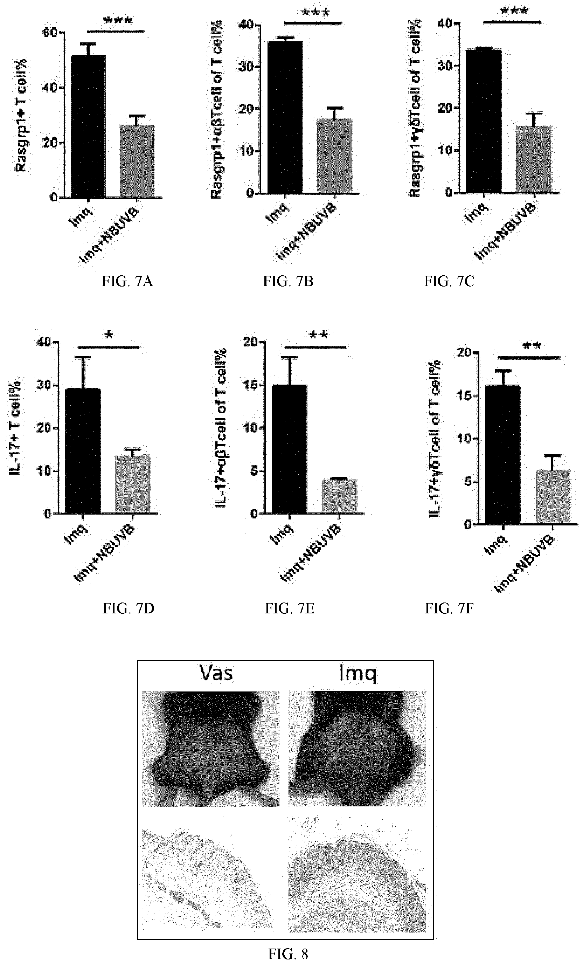

In addition, RASGRP1+T cells and IL-17A+ T cells in the skin tissues of the psoriatic mice treated with the NBUVB radiation are significantly decreased (as shown in FIG. 6 and FIGS. 7A-7F).

The above descriptions indicate that the NBUVB radiation can inhibit the RAS pathway to thereby inhibit T cells generating IL-17, thus alleviating psoriasis-like skin inflammation.

### Embodiment 2 activation of RAS pathways of psoriasis achieved by RASGRP1 to express in T cells of skin tissues

In the embodiment of the invention, skin tissues of psoriatic mice and normal control mice are respectively stained with H&E staining to observe an inflammation of skin lesions of the psoriatic mice. The H&E staining results show that skin capillaries are dilated, epidermal tissues are thickened, and subcutaneous tissues, hair follicles, hair shafts and other skin appendages are infiltrated with inflammatory cells, which indicates the inflammation (as shown in FIG. 8).

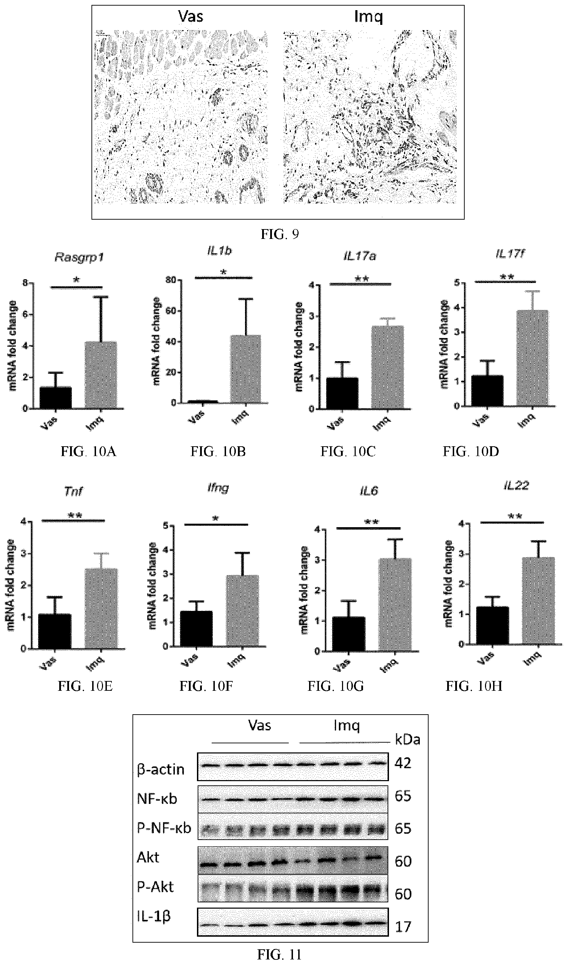

In addition, in order to detect the expression and the location of the RASGRP 1 in the psoriasis, immunohistochemical experiments reveal that the RASGRP1 is mainly expressed in immune cells, and also expressed strongly positive in skin tissues of the psoriatic mice (as shown in FIG. 9).

In addition, the RT-qPCR is used to detect RASGRP1 downstream cytokines, such as ILIb and IL17a. The results show that the expression levels of inflammatory factors in the skin tissues of the psoriatic mice are significantly higher than those of the normal control mice (as shown in FIGS. 10A-10H).

In addition, Western blot detection is used to detect protein expressions of the RASGRP1 downstream pathway gene, and it is found that a total protein level of NF-κB and AKT and a phosphorylated active protein level in the skin tissues of the psoriatic mice are higher than those in the normal control mice, and the active protein level of IL-1β in the skin tissues of the psoriatic mice is higher than that in the normal control mice (as shown in FIG. 11).

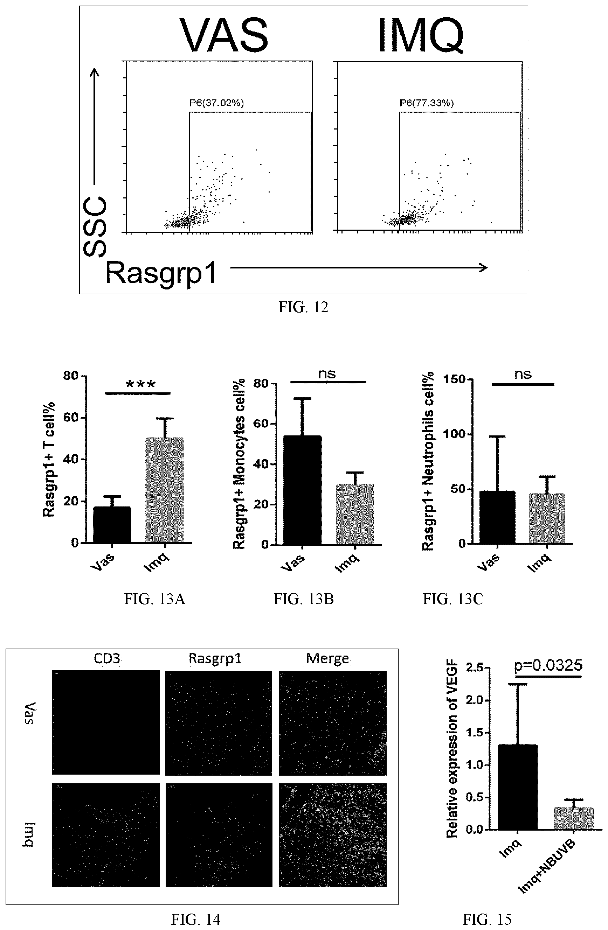

In addition, lymphocytes are respectively extracted from the skin tissues of the normal control mice and the psoriatic mice, and the lymphocytes are grouped with specific labeled antibodies. The results show that there is a significant difference in the expression level of the RASGRP 1 in the skin tissues between the normal control mice and the psoriatic mice when locating in the T cells, but there is no statistical significance in monocytes and neutrophils (as shown in FIG. 12 and FIGS. 13A-13C).

In addition, immunofluorescence detection is used to detect the co-labeling of T cells with RASGRP 1 in the skin tissues of the normal control mice and the psoriatic mice, and the results show that the RASGRP 1 is expressed in the T cells (as shown in FIG. 14).

### Embodiment 3 inhibition of VEGF in skin tissues of psoriatic mice achieved by NBUVB radiation, and secretion of VEGF achieved by epidermal cells

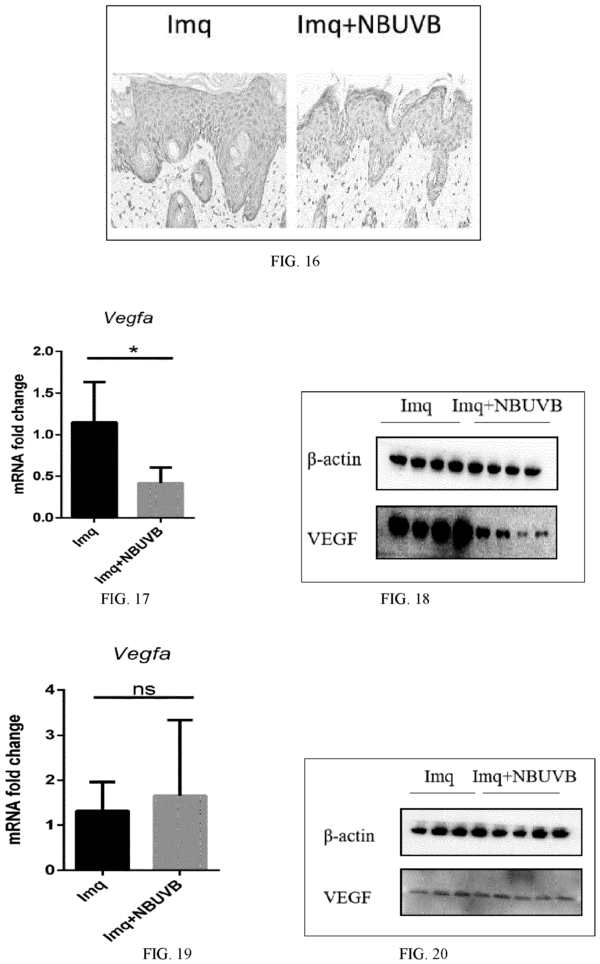

In the embodiment of the invention, the RT-qPCR is used to detect the gene expression of VEGFA (referred to an isoform of the VEGF gene) in back skin tissues of the psoriatic mice and the psoriatic mice treated with the NBUVB radiation. The results show that the NBUVB radiation inhibits the expression of the VEGF factor in the skin tissues of the psoriatic mice (as shown in FIG. 15). Immunohistochemical results show that the VEGF is mainly expressed in the epidermal cells of the skin tissues, while the NBUVB radiation decreases the gene expression of the VEGF in the epidermal cells (as shown in FIG. 16). The epidermal cells and dermal cells of the skin tissues of the psoriatic mice are separated, and the gene expression of the VEGF in the epidermal cells of the psoriatic mice is detected by the RT-qPCR. The results show that the NBUVB radiation inhibits the gene expression of the VEGF in the epidermal cells of the psoriatic mice (as shown in FIG. 17).

In addition, the detection results of Western Blot show that the NBUVB radiation inhibits the protein expression of the VEGFA in the back epidermal cells of the psoriatic mice (as shown in FIG. 18). The results of the RT-qPCR show that the NBUVB radiation does not affect the gene expression of the VEGF gene in the dermal cells of the psoriatic mice (as shown in FIG. 19), and the results of the Western Blot show that the NBUVB radiation does not affect the protein expression of the VEGFA in the back dermal cells of the psoriatic mice (as shown in FIG. 20).

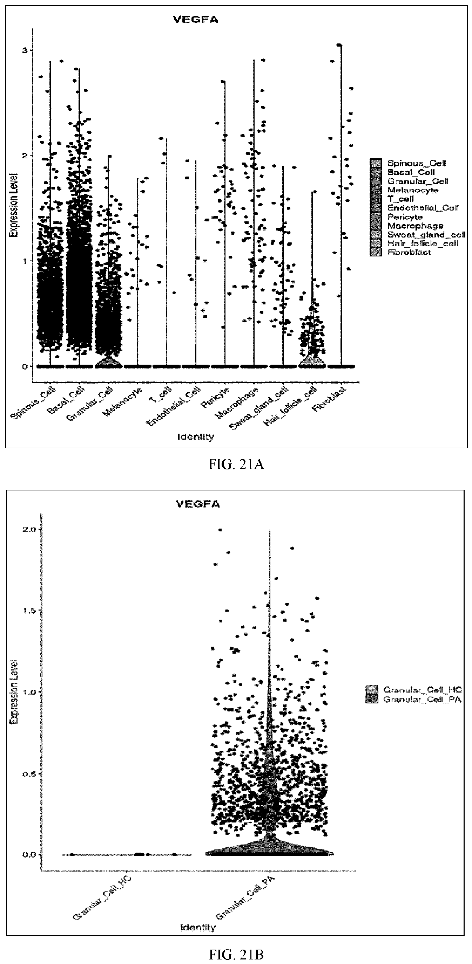

In addition, single-cell sequencing results of human psoriatic lesions show that the VEGFA is mainly secreted by the epidermal cells and the expression of the VEGFA in psoriatic lesions is significantly higher than that in normal tissues in granular layer of skin (as shown in FIGS. 21A-21B).

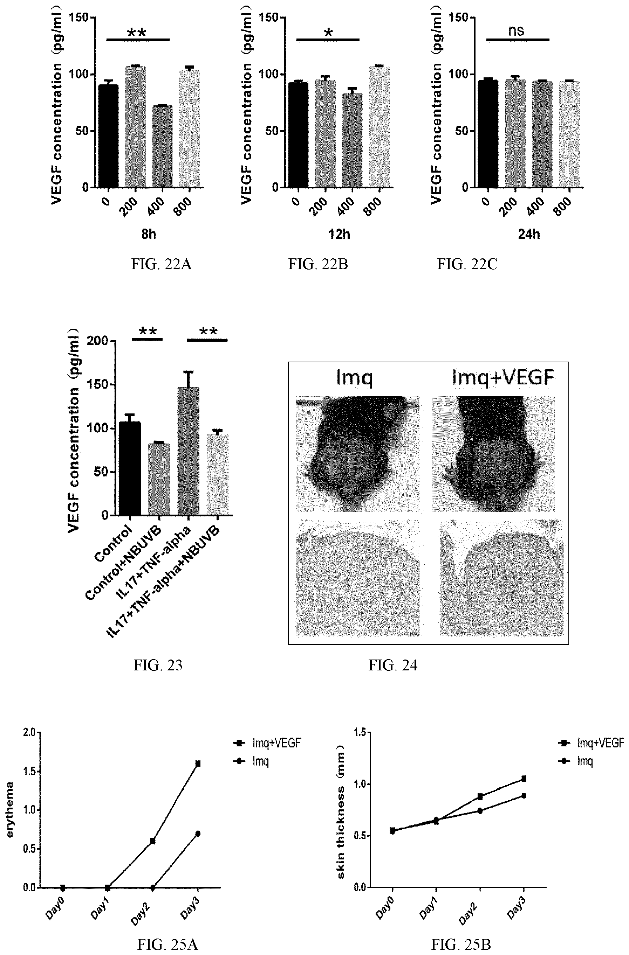

In addition, HaCaT cells (referred to immortalized nontumorigenic human epidermal cells) are irradiated with the NBUVB radiation. Radiation dose gradients are respectively set in 0mj/cm² (referred to energy in per unit area), 200mj/cm², 400mj/cm² and 800mj/cm², and radiation time gradients are respectively set in 8 hours (h), 12 h and 24 h. Changes of the concentration of the VEGF gene is detected by extracting the cell supernatant. The results show that the concentration of the VEGF gene in the cell supernatant significant decreases when the HaCaT cells are irradiated with 400mj/cm² radiation dose for 8 h (as shown in FIGS. 22A-22C).

In addition, the HACAT cells are stimulated by the IL-17A and the TNF-α cytokines, and at the same time irradiated by 400mj/cm² NBUVB radiation dose. Elisa detection is used to detect the concentrations of the VEGFA in the cell supernatants that are not irradiated by the NBUVB radiation and the concentrations of the VEGFA in the cell supernatants that are irradiated by the NBUVB radiation. It is found that the concentration of the VEGFA secreted by the cells that are irradiated by the NBUVB radiation is significantly lower than that secreted by the cells that are not irradiated by the NBUVB radiation (as shown in FIG. 23).

### Embodiment 4 aggravation of phenotype of psoriatic mice achieved by subcutaneously injected with VEGF, and alleviation of phenotype of psoriatic mice achieved by VEGFR inhibitors

In the embodiment of the invention, psoriatic mice coated with VEGFR inhibitors are constructed as follows: dissolving the VEGFR inhibitor SU5205 in dimethyl sulfoxide (DMSO) solution to prepare stock solution, diluting the stock solution with cyclodextrin, and coating the stock solution with a concentration of 10kg/kg/d (referred to a daily dosage per kilogram of body weight of the psoriatic mice) on the psoriatic mice. 7-8-week wild-type mice are taken with back hair shaved and an exposed skin area of 2 cm×2 cm. 150µL of the VEGFR inhibitor solution is coated on the skin surface. The normal control mice are coated with control reagent (with 150µL of DMSO and cyclodextrin). The psoriatic mice and the normal control mice are respectively coated with Imiquimod (IMQ) cream or control reagent VAS on the exposed skin in 4 h later of the above operation.

In the embodiment of the invention, the psoriatic mice subcutaneously injected with VEGF are constructed as follows: dissolving VEGF165 protein in DMSO solution to prepare stock solution, diluting the stock solution with cyclodextrin, and smearing the mice with the stock solution with a concentration of 10kg/kg/d. 7-8-week wild-type mice are taken with back hair shaved and an exposed skin area of 2 cm×2 cm. 100µL of the VEGF165 solution is injected subcutaneously the skin surface of the mice. The normal control mice are injected with control reagent subcutaneously (with 100µL of DMSO and cyclodextrin). The psoriatic mice subcutaneously injected with VEGF165, and the normal control mice are respectively smeared IMQ cream or control reagent VAS on the exposed skin in 4 h later of the injections.

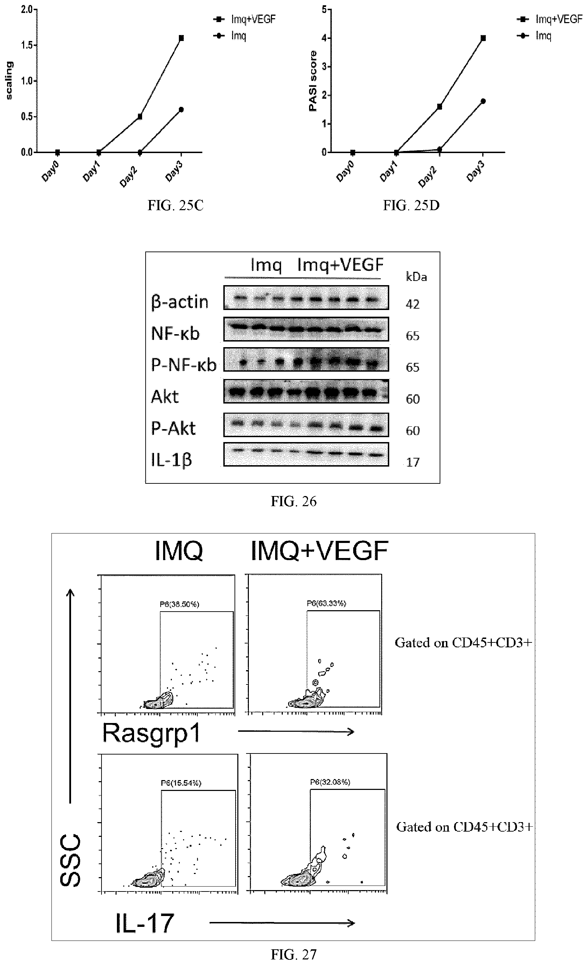

In the embodiment of the invention, the IMQ-induced psoriatic mice based on VEGF overexpression are constructed according to the above method. The results of H&E staining and PASI scores of skin sections show that, compared with the IMQ-induced mice, the psoriasis-like phenotypes of the psoriatic mice subcutaneously injected with VEGF165 are significantly aggravated, which is reflected by increases of dorsal skin thickness, erythema, and scaling (as shown in FIG. 24 and FIGS. 25A-25D).

In addition, Western blot detection is used to detect the protein expressions of the RASGRP1 downstream pathway gene, and it is found that the active protein levels of the NF-κB and the phosphorylated AKT in the skin tissues of the psoriatic mice with VEGF overexpression are significantly increased, and the active protein level of the IL-1β is also significantly higher than that of the IMQ-induced mice (as shown in FIG. 26).

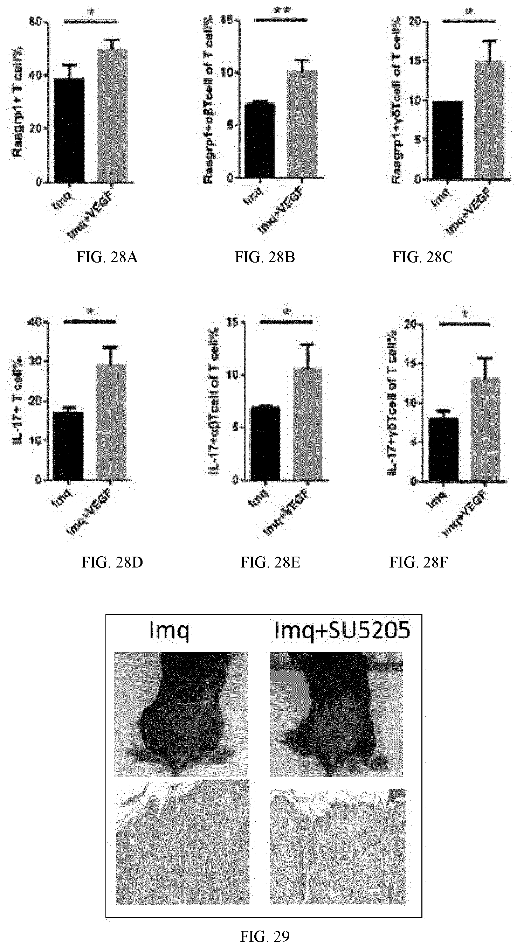

In addition, the RASGRP1+T cells and IL-17A+T cells in the skin tissues of the psoriatic mice with VEGF overexpression increase significantly (as shown in FIG. 27 and FIGS. 28A-28F), indicating that the VEGF overexpression in the skin tissues can activate the secretion of the RASGRP 1 and relative downstream inflammatory factors to thereby regulate T cells generating IL-17, and then affecting the psoriasis-like skin inflammation.

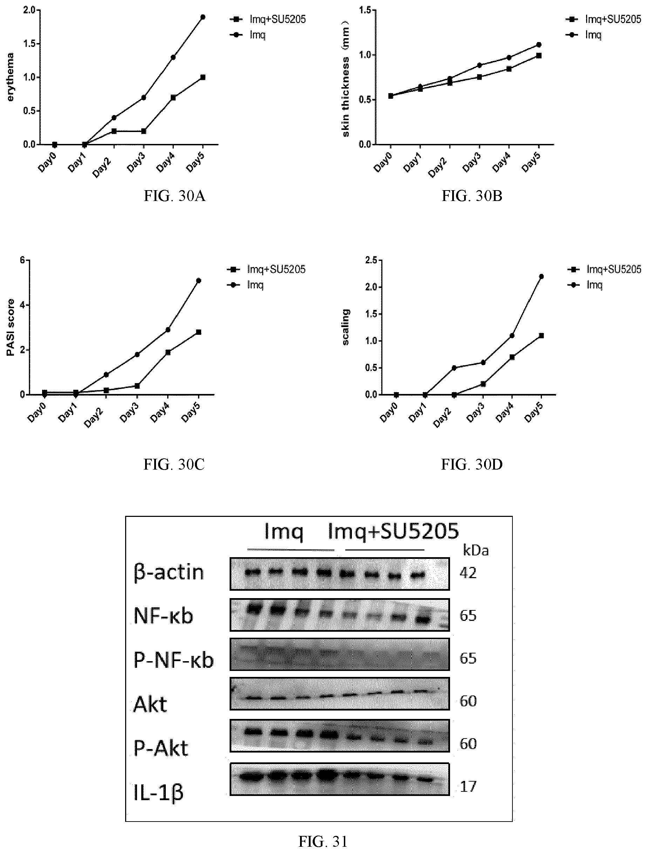

In addition, in order to further prove the reaction mechanism of the VEGF in the psoriasis, skin tissues of the mice are smeared with VEGF receptor inhibitor SU5205 to construct a psoriatic mice model with inhibiting VEGFR in the skin tissues. The results of H&E staining of skin sections and PASI scores show that, compared with the IMQ-induced mice, the psoriasis phenotypes of the psoriatic mice with skin smeared with SU5205 are significantly reduced, which is reflected by reductions of dorsal skin thickness, erythema, and scaling (as shown in FIG. 29 and FIGS. 30A-30D).

In addition, Western blot detection is used to detect the protein expressions in the RASGRP1 downstream pathway gene, and it is found that the active protein levels of the NF-κB and the phosphorylated AKT in the skin tissues of the psoriatic mice with the skin smeared with the SU5205 are significantly reduced, and the active protein level of the IL-1β is also significantly lower than that of the IMQ-induced mice (as shown in FIG. 31).

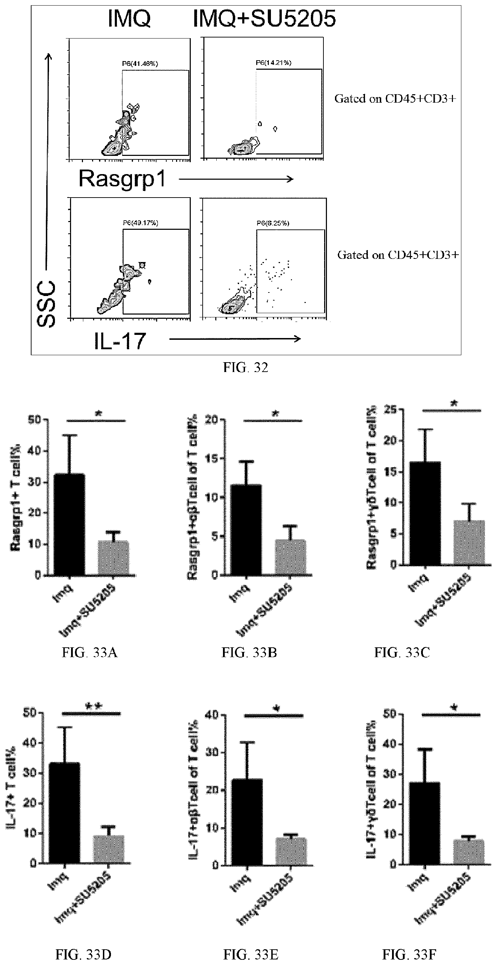

In addition, Rasgrp1+T cells and IL-17A+T cells in the skin tissues of the psoriatic mice with the skin smeared with the SU5205 decrease significantly (as shown in FIG. 32 and FIGS. 33A-33F).

The above descriptions indicate that inhibiting VEGFR in the skin tissues can reduce the secretion of the RASGRP 1 and the relative downstream inflammatory factors to thereby regulate T cells generating IL-17, and thus alleviating the psoriasis-like skin inflammation.

### Embodiment 5 activation of Jurkat cells promoted by VEGF, and secretion changes of RAS downstream pathway inflammatory factors caused by RASGRP1 overexpression and RASGRP1 knockdown

In the embodiment of the invention, the Jurkat cells are infected with RASGRP1 lent virus, cell models with stable RASGRP1 overexpression and RASGRP1 knockdown are respectively constructed. VEGF cytokines are used to stimulate the Jurkat cells to detect whether the VEGF can promote the activation of the Jurkat cells and detect whether the RASGRP1 overexpression and the RASGRP1 knockdown can cause the secretion changes of the RAS pathway downstream inflammatory factors.

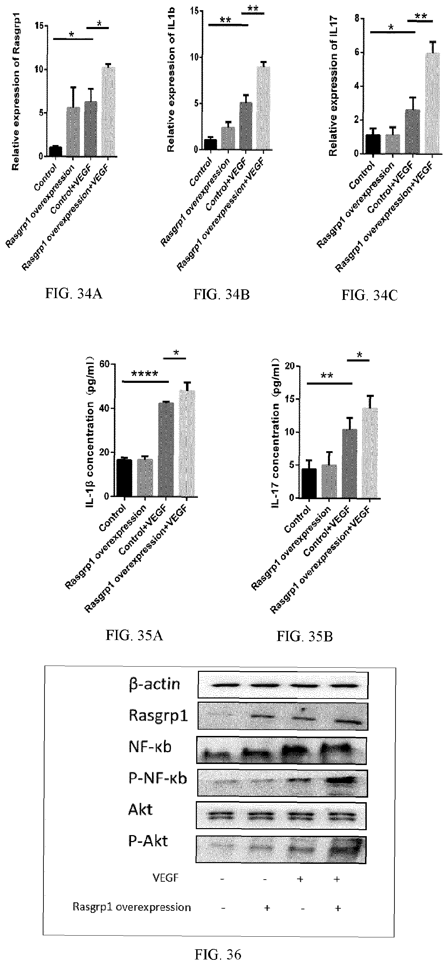

In addition, the results of the RT-qPCR show that the secretion of the RASGRP1 and the relative downstream inflammatory factors in the Jurkat cells stimulated by the VEGF significantly increase. Under the stimulation of the VEGF gene, the RASGRP1 overexpression significantly increases the secretion of the RAS pathway downstream inflammatory factors (as shown in FIGS. 34A-34C).

In addition, the Elisa detection results show that the concentrations of the IL-1β and the IL-17 in the cell supernatants of the Jurkat cells with RASGRP 1 overexpression stimulated by the VEGF are significantly higher than those in the normal control mice (as shown in FIGS. 35A-35B). The results of Western blot show that the active protein levels of the NF-κB and the phosphorylated AKT of the RASGRP1 and relative pathway downstream in the Jurkat cells are significantly increased under the stimulation of the VEGF. Under the stimulation of the VEGF, the active protein levels of the NF-κB and the phosphorylated AKT are significantly higher in the RASGRP1 overexpression cells, where the active protein level of the IL-1β is also significantly higher than that of the normal control mice (as shown in FIG. 36).

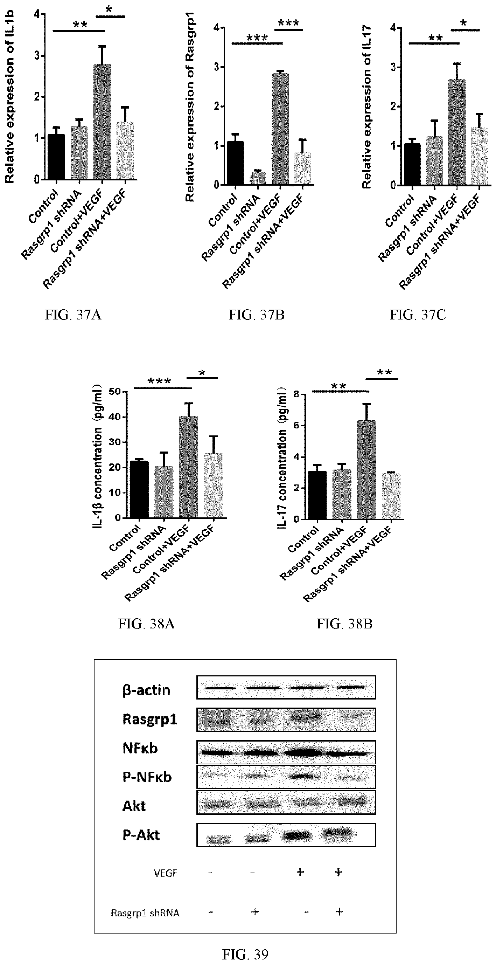

In addition, the results of the RT-qPCR show that the expression levels of the IL1b and the IL17 transcriptome in the Jurkat cells with knocking down the RASGRP1 stimulated by the VEGF are significantly lower than those in the cells without knocking down the Rasgrp1 (as shown in FIGS. 37A-37C).

In addition, the results of the Elisa detection show that the secretion concentrations of the IL-1β and the IL-17 in the cell supernatants with knocking down the RASGRP1 stimulated by the VEGF are significantly lower than those in the normal control mice (as shown in FIGS. 38A-38B). The results of Western blot show that under the stimulation of the VEGF, the active protein levels of the NF-κB and the phosphorylated AKT in the cells with knocking down the RASGRP 1 are significantly reduced, and the active protein level of the IL-1β is also significantly lower than that of the normal control mice (as shown in FIG. 39).

The above descriptions indicate that the VEGF can promote the activation of the Jurkat cells, and the RASGRP1 overexpression and the RASGRP1 knockdown can cause secretion changes of the RAS pathway downstream inflammatory factors.

### Embodiment 6 aggravation of phenotype of psoriatic mice caused by RASGRP1 overexpression in skin tissues

In the embodiment of the invention, psoriasis mice subcutaneously injected with RASGRP1 overexpression adeno-associated virus are constructed as follows: mice are injected with RASGRP1 adeno-associated virus subcutaneously, and an amount of the adeno-associated virus injected to each mouse is 5×10¹¹ V.G. The adeno-associated virus is diluted with phosphate buffered solution (PBS), and the mice are divided into eight groups, four normal control groups and four injection groups at four time points. The four normal control groups are injected with the PBS. The eight groups of mice are respectively sacrificed at the 7^{th}-day, the 14^{th}-day, the 21^{st}-day and the 28^{th}-day after the injections to extract the skin tissues to generate fluorescence quantitative PCR experiments to detect the concentration of the RASGRP1 in the skin tissues of the mice.

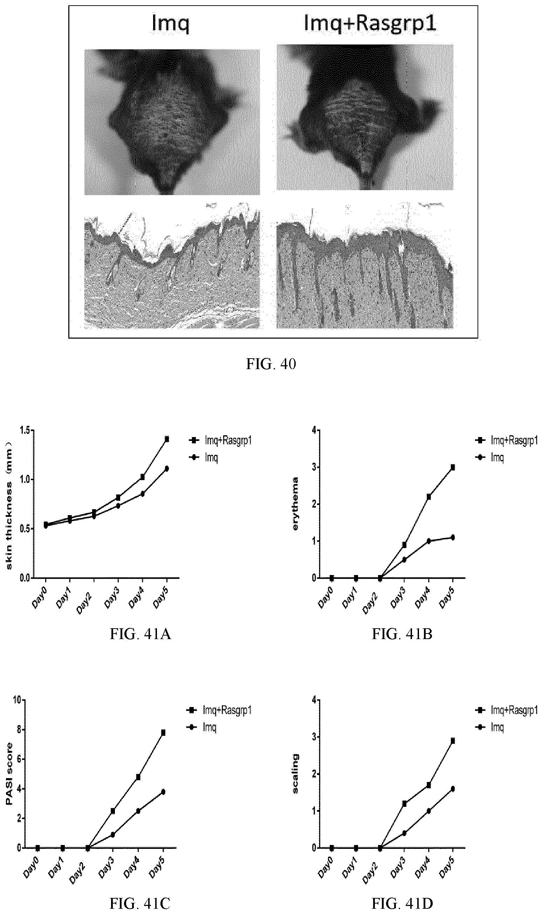

In the embodiment of the invention, in order to further verify the reaction mechanism of the RASGRP1 in the psoriasis, IMQ-induced psoriatic mice based on RASGRP1 overexpression are constructed according to the above method. The results of H&E staining and PASI scores of skin sections show that, compared with the WT IMQ-induced mice, the psoriasis-like phenotype of the psoriatic mice with RASGRP1 overexpression are significantly aggravated, which is reflected by increases of dorsal skin thickness, erythema, and scaling (as shown in FIG. 40 and FIGS. 41A-41D).

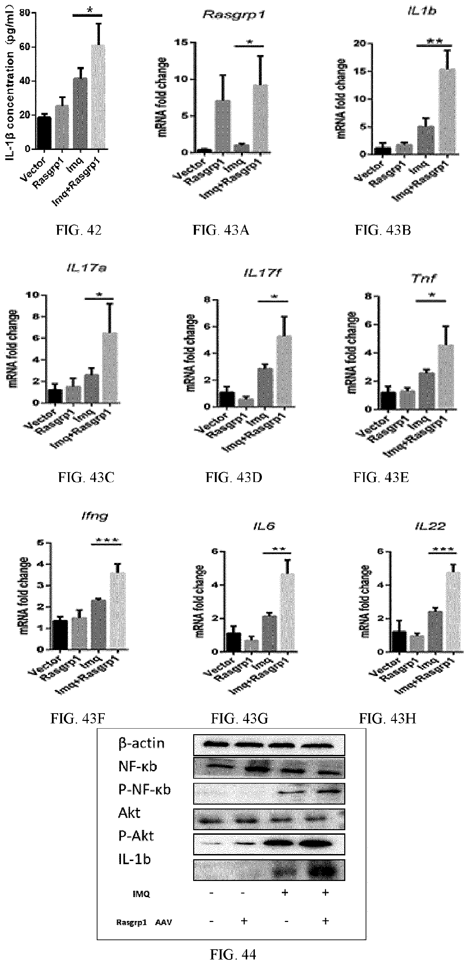

In addition, the results of Elisa detection show that the concentration of IL-1β in the skin tissues of the psoriatic mice with RASGRP1 overexpression are significantly higher than that of the normal control groups (as shown in FIG. 42).

In addition, the RT-qPCR detects the RASGRP1 and relative downstream cytokines, and it is found that the expression levels of the RASGRP1, IL1b, IL17a and relative inflammatory genes in the skin tissues of the psoriatic mice with RASGRP1 overexpression are significantly increased (as shown in FIGS. 43A-43H). In addition, Western blot detects protein expressions of the RASGRP1 downstream pathway gene, and it is found that the active protein levels of the NF-κB and the phosphorylated AKT in the skin tissues of the psoriatic mice with RASGRP1 overexpression are significantly increased, and the active protein level of the IL-1β is also significantly higher than that of the normal control group (as shown in FIG. 44).

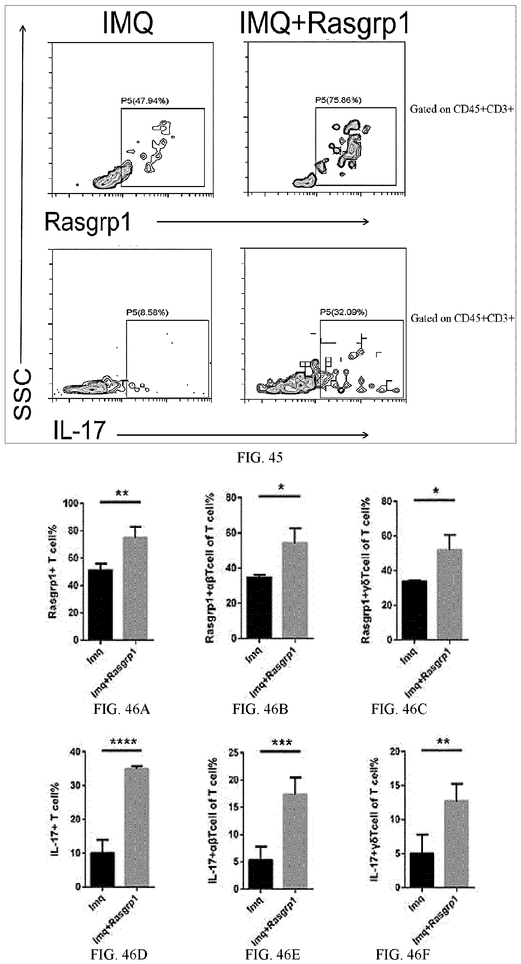

In addition, the IL-17A+T cells in the skin tissues of the psoriatic mice with RASGRP1 overexpression increase significantly (as shown in FIG. 45 and FIGS. 46A-46F).

The above descriptions indicate that the RASGRP1 overexpression in the skin tissues can activate the secretion of RASGRP1 downstream inflammatory factors to thereby regulate T cells generating IL-17, thus affecting the psoriasis-like skin inflammation.

### Embodiment 7 alleviation of phenotype of psoriatic mice with RASGRP1 overexpression achieved by NBUVB radiation

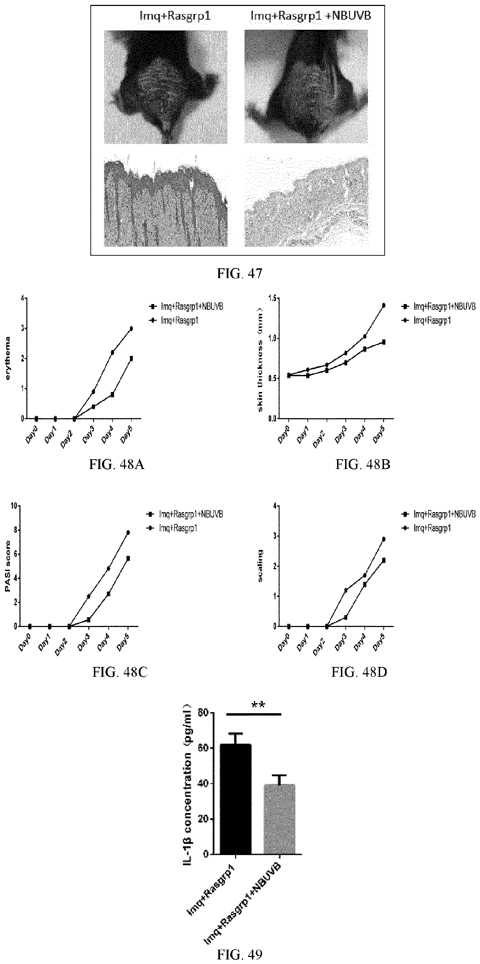

In the embodiment of the invention, in order to further verify the reaction mechanism of the NBUVB radiation in the psoriasis, the psoriatic mice with RASGRP1 overexpression are treated with the NBUVB radiation. The results of H&E staining and PASI scores of skin sections show that, compared with psoriatic mice with RASGRP1 overexpression, the phenotypes of the psoriatic mice with RASGRP1 overexpression treated with NBUVB radiation are significantly reduced, which is reflected by reductions of dorsal skin thickness, erythema, and scaling (as shown in FIG. 47 and FIGS. 48A-48D).

In addition, Elisa detection results show that the concentration of the IL-1β in the skin tissues of the psoriatic mice with RASGRP 1 overexpression treated with NBUVB radiation is significantly lower than that of the psoriatic mice with RASGRP1 overexpression (as shown in FIG. 49).

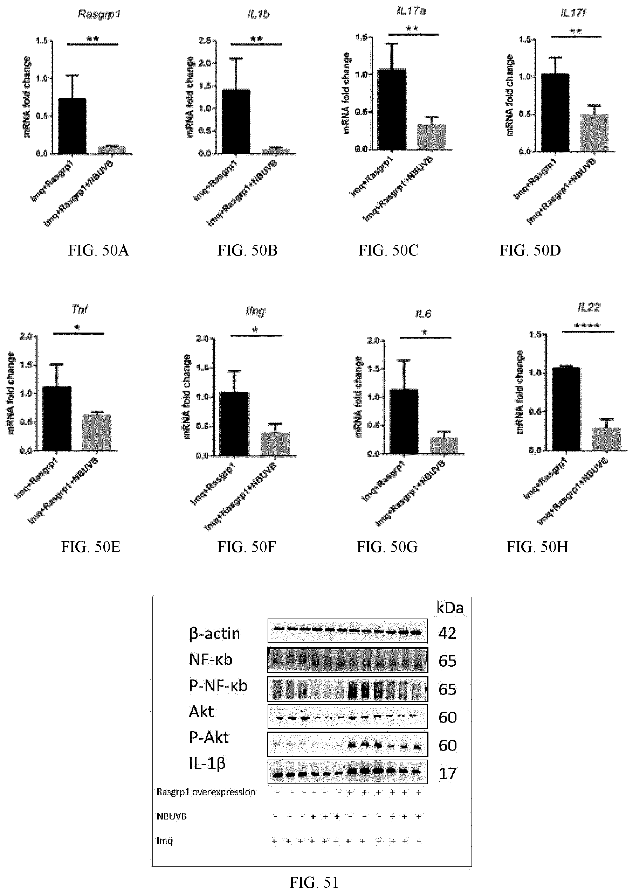

In addition, the RT-qPCR detects the RASGRP1 gene and relative downstream cytokines, such as ILIb and IL17a, and it is found that the gene expression levels of the RASGRP1, the ILIb and IL17a in the skin tissues of the psoriatic mice with RASGRP1 overexpression treated with NBUVB radiation are significantly reduced (as shown in FIGS. 50A-50H).

In addition, Western blot detection is used to detect protein expression of the RASGRP1 downstream pathway genes, and it is found that the total active protein levels of the NF-κB and the phosphorylated AKT in the skin tissues of the psoriatic mice with RASGRP1 overexpression treated with NBUVB radiation are significantly reduced, and the active protein level of IL-1β is also significantly lower than that of the psoriatic mice with RASGRP1 overexpression (as shown in FIG. 51).

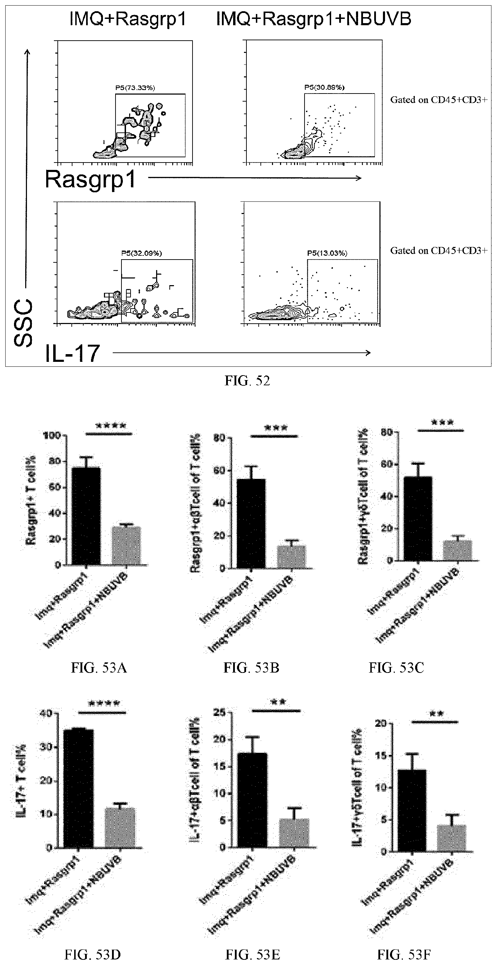

In addition, the IL-17A+T cells in the skin tissues of the psoriatic mice with RASGRP1 overexpression treated with NBUVB radiation significantly reduce (as shown in FIG. 52 and FIGS. 53A-53F).

The above descriptions indicate that the NBUVB radiation can inhibit the over-expressed RAS pathway in the skin tissues to thereby inhibit downstream inflammatory factors and inhibit T cells generating IL-17, thus alleviating psoriasis-like skin inflammation.

### Embodiment 8 alleviation of phenotype of psoriatic mice achieved by knocking out RASGRP1

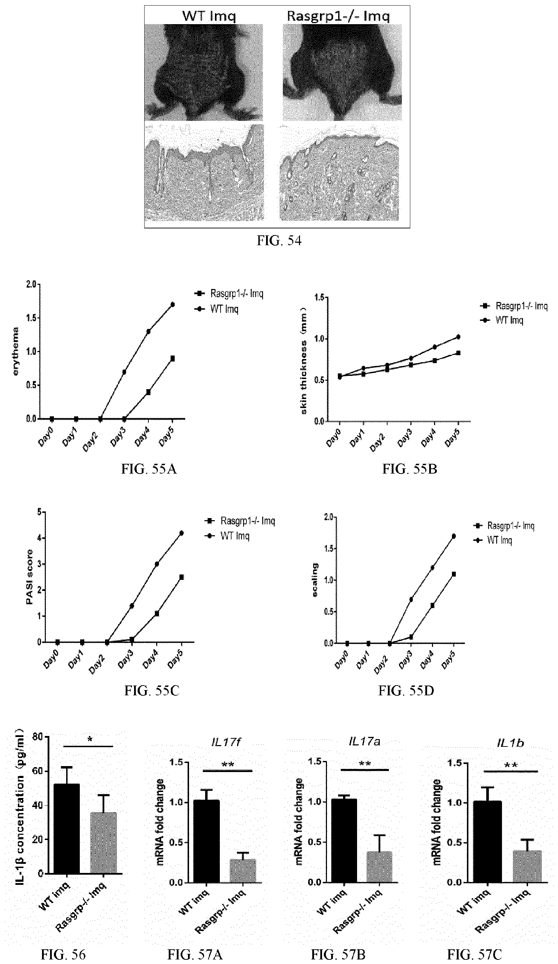

In the embodiment of the invention, in order to further verify action mechanism of the RASGRP1 in the psoriasis, mice with knocking out the RASGRP1 are constructed to observe whether knocking out the RASGRP1 can affect the phenotypes of the psoriatic mice. The results of H&E staining of skin sections and PASI scores show that compared with the wild-type (WT) imiquimod-induced (IMQ-induced) mice, the phenotypes of the psoriatic mice with knocking out the RASGRP1 are significantly alleviated, which is reflected by reductions of dorsal skin thickness, erythema, and scaling (as shown in FIG. 54 and FIGS. 55A-55D).

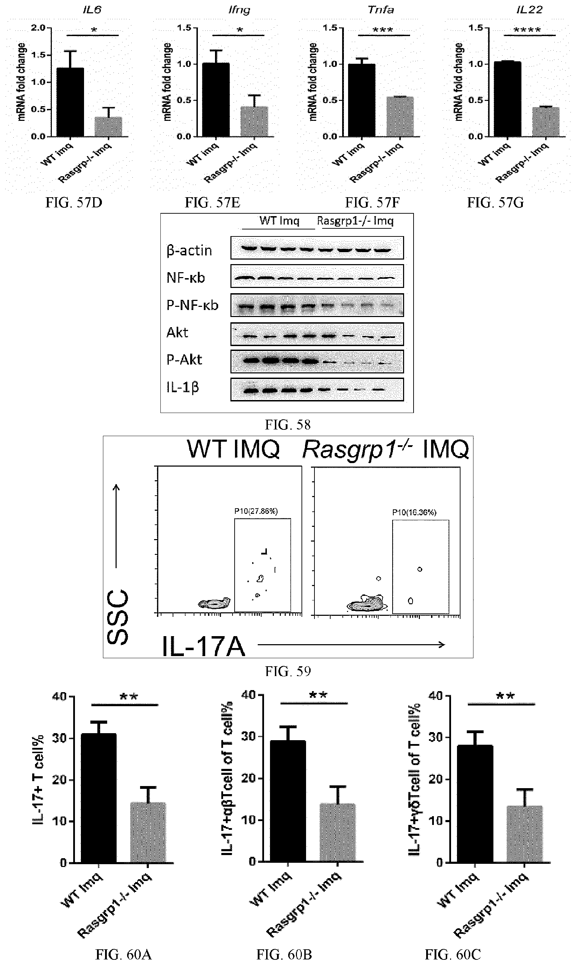

In addition, Elisa detection results show that the concentration of IL-1β in the skin tissues of the psoriatic mice with knocking out the RASGRP1 is significantly lower than that of the WT IMQ-induced mice (as shown in FIG. 56). The RT-qPCR is used to detect RASGRP1 downstream cytokines, and it is found that, compared with the WT IMQ-induced mice, the expression levels of the IL1b, IL17a and relative inflammatory genes in the skin tissues of the psoriatic mice with knocking out the RASGRP1 are significantly reduced (as shown in FIGS. 57A-57G). In addition, Western blot detection is used to detect the protein expressions of the RASGRP 1 downstream pathway genes, and it is found that, compared with the WT IMQ-induced mice, the active protein levels of the NF-κB and the phosphorylated AKT in the skin tissues of the psoriatic mice with knocking out the RASGRP1 are significantly reduced, and the active protein level of the IL-1β is also significantly lower than that of the WT IMQ-induced mice (as shown in FIG. 58).

In addition, compared with the WT IMQ-induced mice, the IL-17A+T cells in the skin tissues of the psoriatic mice with knocking out the RASGRP1 decrease significantly (as shown in FIG. 59 and FIGS. 60A-60C).

The above descriptions indicate that knocking out the RASGRP1 in the skin tissues can inhibit the secretion of RASGRP1 downstream inflammatory factors to thereby inhibit T cells generating IL-17, and thus alleviating psoriasis-like skin inflammation.

Finally, it should be noted that the above embodiments only aim to describe the technical schemes of the invention but not to limit the invention. Although the illustrated embodiments describe the invention in detail, it should be understood by those skilled in the related field that modification or equivalent replacement made to the technical schemes of the invention without departing from the purpose and the scope of the technical schemes of the invention shall be covered by the scope of the protection of the invention.

## Claims

1. An application of a vascular endothelial growth factor receptor (VEGFR) gene expression inhibitor, comprising:
preparing a preparation by using the VEGFR gene expression inhibitor for treating psoriasis; or taking the VEGFR gene expression inhibitor as a preparation for treating psoriasis.

2. An application of a renin-angiotensin system guanyl releasing protein 1 (RASGRP1) gene expression inhibitor, comprising:
preparing a preparation by using the RASGRP 1 gene expression inhibitor for treating psoriasis; or taking the RASGRP 1 gene expression inhibitor as a preparation for treating psoriasis.

3. The application according to claim 2, further comprising:
knocking out or knocking down a RASGRP1 gene by using the RASGRP1 gene expression inhibitor.

4. The application according to any one of claims 1-3, wherein the preparation comprises one of a chemical reagent and a biological reagent.

5. The application according to claim 4, wherein the preparation is in a form of one of an ointment, powder, and an aqueous solution.

6. An application of narrow-band ultraviolet B (NBUVB) radiation, comprising:
inhibiting an expression of a VEGF gene by using the NBUVB radiation.

7. An application of narrow-band ultraviolet B (NBUVB) radiation, comprising:
inhibiting an expression of a RASGRP 1 gene by using the NBUVB radiation.

8. An application of narrow-band ultraviolet B (NBUVB) radiation, comprising:
inhibiting T-lymphocytes generating interleukin-17 (IL-17) by using the NBUVB radiation.

9. A method for inhibiting T-lymphocytes generating IL-17, comprising:
performing one of NBUVB radiation, knocking out or knocking down a VGEF gene, and knocking out or knocking down a RASGRP1 gene, thereby inhibiting the T-lymphocytes generating the IL-17.
